(19) Europäisches Patentamt
European Patent Office
Office européen des brevets

(11) Publication number: 0 005 015 B1

(12) EUROPEAN PATENT SPECIFICATION

(45) Date of publication of patent specification: 29.10.86

(51) Int. Cl.$^4$: A 61 K 31/52

(21) Application number: 79300516.6

(22) Date of filing: 29.03.79

# (54) Use of a xanthine derivate in the manufacture of a medicament.

(30) Priority: 22.04.78 GB 1600878

(43) Date of publication of application:
31.10.79 Bulletin 79/22

(45) Publication of the grant of the patent:
29.10.86 Bulletin 86/44

(84) Designated Contracting States:
BE CH DE FR GB IT LU NL SE

(56) References cited:
DE-A-2 462 367
GB-A-1 441 562
GB-A-1 470 220

Acta anaesth. scand. (1980), 24, p. 347-353

Pflügers Arch. (1973), 345, p. 335-346

(73) Proprietor: BEECHAM - WUELFING GmbH & Co. KG
Stresemannallee 6 P.O. Box 25
D-4040 Neuss (DE)

(72) Inventor: Goring, Joachim Ewald
Sauerweinstrasse 25
D-3212 Gronau (Leine) (DE)
Inventor: Ochlich, Peter Paul
Wilhelm-Raabe-Strasse 6
D-3212 Gronau (Leine) (DE)

(74) Representative: Hesketh, Alan, Dr.
European Patent Attorney
Beecham Pharmaceuticals
Great Burgh Yew Tree Bottom Road
Epsom, Surrey, KT18 5XQ (GB)

EP 0 005 015 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

The present invention relates to the use of 1,3-di-n-butyl-7-(2-oxopropyl)- xanthine in the manufacture of a medicament.

British Patent Specification No. 1441562 referred to the compounds of the formula (I):

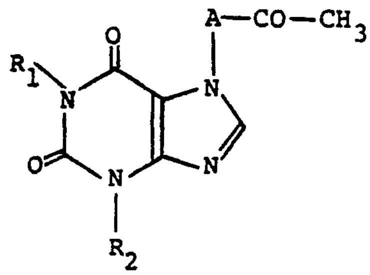

(I)

wherein $R_1$ and $R_2$ which may be the same or different, each represents a straight-chain or branched-chain alkyl radical of 2 to 6 carbon atoms, or a cyclohexyl, alkoxyalkyl or hydroxyalkyl radical, and A represents a hydrocarbon radical having up to 4 carbon atoms which may be substituted by a methyl group. The compounds of the formula (I) were described as effective in increasing the blood flow through skeletal muscles while at the same time showing low toxicity. The compound of the formula (I) said to be preferred was that wherein $R_1$ is an n-butyl group, $R_2$ is an n-butyl group and A is a $CH_2CH_2$ group. That compound was shown to be highly effective. German Patent Application No. 2462367 indicates that the compounds of formula (I) may in general be employed as unit doses of about 200—600 mgs so that they would be expected to be used at a similar dose to known agents such as pentoxyphylline. The published low oral toxicity of xanthines such as pentoxyphylline and the compound of the formula (I) where $R_1$ and $R_2$ are n-butyl groups and A is a $CH_2CH_2$ group means that such high doses are acceptable.

It has now been discovered that 1,3-di-n-butyl-7-(2-oxopropyl)xanthine, which falls within formula (I) and is disclosed in GB—A—1441562 (see Table VIII) and in DE—A—2462367 (see Table XI), is extremely potent in increasing oxygen tension and contractility in ischaemic skeletal muscle. These properties reflect an improvement in the metabolic status of the tissue which in turn makes the compound of great potential use as an agent for the treatment of peripheral vascular disease such as intermittent claudication.

Accordingly, in one aspect the present invention provides the use of 1,3-di-n-butyl-7-(2-oxopropyl) xanthine for the manufacture of a medicament for increasing oxygen tension and contractility in ischaemic skeletal muscle.

In another aspect, the present invention provides the use of 1,3-di-n-butyl-7-(2-oxopropyl)xanthine for the manufacture of a medicament for the treatment of peripheral vascular disease, such as intermittent claudication.

This compound has a low acute toxicity so that the conventional high doses would have been expected to be used in the clinic. However, the extremely high potency of this compound allows its use in surprisingly low dose.

Thus in a preferred aspect, the invention provides the use of 1,3-di-n-butyl-7-(2-oxopropyl)xanthine for the manufacture of a medicament as defined above, wherein the medicament is a pharmaceutical composition which comprises 1 to 30 mg of 1,3-di-n-butyl-7-(2-oxopropyl)xanthine and a pharmaceutically acceptable carrier therefor.

The composition may contain from 2 to 25 mg of 1,3-di-n-butyl-7-(2-oxopropyl)xanthine, from 2.5 to 20 mg or from 5 to 15 mg.

Thus suitable compositions may contain about, for example, 2.5, 5, 7.5, 10, 12.5, 15, 17.5 or 20 mg of 1,3-di-n-butyl-7-2-oxopropyl)xanthine.

The compositions may be administered one or more times a day so that the daily dose is in the region of 2.5—90 mg, and more usually 5—50 mg, for example about 10—40 mg. The composition is often administered twice or three times a day.

Generally the compositions will be adapted for administration by injection or for oral administration.

Although 1,3-di-n-butyl-7-(2-oxopropyl)xanthine is only sparingly soluble in aqueous media the enhanced potency of the compound renders it suitable for use in injectable solutions, for example in aqueous solution. The injectable compositions may consist essentially of said sterile, pyrogen free 1,3-di-n-butyl-7-(2-oxopropyl)xanthine, for example sealed into a vial or ampoule or the like. Other suitable injectable compositions may comprise said sterile material in admixture with suspending agents, preserving agents or the like. Such compositions may be made up for injection on admixture with sterile water or saline or the like. In general the volume to be injected will be from 0.5 to 2 mls, for example 1 ml.

Suitably the injectable composition will contain slightly less than the maximum orally administrable composition, for example from 1 to 25 mg of 1,3-di-n-butyl-7-(2-oxopropyl)xanthine. More suitably the injectable composition will contain 2 to 20 mg and preferably 2.5 to 15 mg of 1,3-di-n-butyl-7-(2-oxopropyl)xanthine; for example about 5, 7.5, 10 or 12.5 mgs of said agent.

Particularly favoured compositions are those adapted for oral administration since they are more convenient for general use. Such dosage forms include tablets and capsules and the like. The dosage units may contain such conventional agents as fillers (diluents), lubricants, binders, disintegrants, colourants, flavourings, surface active agents, preservatives, buffering agents and the like.

Suitable fillers for use include cellulose, manitol, lactose and other similar agents. Suitable disintegrants include starch, polyvinylpolypyrrolidone and starch derivatives such as sodium starch glycollate and the like. Suitable lubricants include stearic acid, magnesium stearate, magnesium lauryl sulphate and the like.

Since 1,3-di-n-butyl-7-(2-oxopropyl)xanthine is a medicament of high potency the solid orally administrable unit dosage from may be small, for example under 80 mgs in weight, but for patient convenience it is usual to formulate the composition in such a manner that it weighs about 80—600 mgs, in total, for example about 100—400 mgs. This means that frequently relatively large proportions of a filler is employed. Thus formation of unit dosage forms will be simple since the skilled worker may select fillers or other agents of known physical properties to prepare the composition in conventional manner as the actual effect of the small quantity of 1,3-di-n-butyl-7-(2-oxopropyl)xanthine is slight.

The oral compositions may be prepared by conventional methods of blending, filling, tabletting or the like. Repeated blending operations may be used to distribute the active agent throughout those compositions employing large quantities of fillers. Such operations are of course conventional in the art.

The following Examples illustrate the invention:

## Example 1

1,3-Di-n-butyl-7-(2-oxopropyl)xanthine, magnesium stearate and microcrystalline cellulose were blended together and passed through a 40 mesh sieve (UK). The mixture was tabletted on a conventional rotary machine to produce a batch of 5000 tablets fo the following composition.

| | |
|---|---|
| 1,3-D-n-butyl-7-(2-oxopropyl)xanthine: | 10 mg |
| magnesium stearate: | 0.2 mg |
| microcrystalline cellulose: | 189.8 mg |

## Example 2

1,3-Di-n-butyl-7-(2-oxopropyl)xanthine, sodium lauryl sulphate, lactose and sodium starch glycollate were blended together and passed through a 40 mesh sieve (UK). The mixture was tabletted on a conventional rotary machine to produce a batch of 5000 tablets of the following composition:

| | |
|---|---|
| 1,3-Di-n-butyl-7-(2-oxopropyl)xanthine: | 5 mg |
| magnesium lauryl sulphate: | 0.1 mg |
| lactose | 103 mg |
| sodium starch glycollate: | 1.9 mg |

## Example 3

a) 5.0 g of 1,3-di-n-7-(2-oxopropyl)xanthine were mixed with 51.04 g microcrystalline cellulose, passed through a 0.8 mm sieve with 91.35 g lactose and 2.61 g hydrogenated castor oil and mixed in a cubic mixer. The mixture was pressed into tablets of 150 mg with a single punch of diameter 7 mm each tablet containing 5 mg of 1,3-di-n-butyl-7-(2-oxopropyl)xanthine.

b) The mixture prepared as above was filled into capsules size 3, so that each capsule contained 150 mg of the mixture.

## Example 4

a)10.0 g of 1,3-di-n-butyl-7-(2-oxopropyl)xanthine were mixed with 189.8 g microcrystalline cellulose, passed through a 0.8 mm sieve with 0.2 g magnesium stearate and mixed in a cubic mixer. The mixture was pressed into tablets of 200 mg with a single punch of diameter 8 mm, each tablet containing 10 mg of 1,3-di-n-butyl-7-(2-oxopropyl) xanthine.

b) The mixture prepared as above was filled into capsules size 0, so that each capsule contained 200 mg of the mixture.

*Illustration of Pharmacological Effectiveness of Xanthine Derivatives*

In the following illustrations 1,3-di-n-butyl-7-(2-oxopropyl)xanthine will be referred to as "Compound A", pentoxyphylline will be referred to as "Compound B" and 1,3-di-butyl-7-(3-oxobutyl)xanthine will be referred to as "Compound C".

*Investigation of the effects of Compounds A,B and C upon oxgyen tension and contractility of ischaemic skeletal muscle of cats*

Methodology:

Anaesthesia and administration of compounds:

Cats of either sex were anaesthetized with urethane/chloralose (120/60 mg/kg i.p. In the course of the

experiments, pentobarbital was injected intravenously (V. antebrachii ceph.) via a plastic tube. The intraduodenal (i.d.) application of compound weas conducted by means of a plastic catheter, which was inserted into the duodenum.

a)$pO_2$-measurements:

$pO_2$ was measured polarographically using Pt-needle electrodes (modified according to Bäumgartl and Lübbers, 1973). The reference electrode, and Ag/AgCl system was sputtered on the glass mantle of the cathode, or it was a separate electrode. The cathode was coated with a polystyrol and a collodium membrane. A constant voltage supply provided a polarizing voltage between 600 and 800 mV. The reducing current was measured by a nano-ampere amplifying meter. The cathode was inserted in the tissue by means of a motor-driven micromanipulator.

Characteristics of $pO_2$-elecrodes

| characteristics | $pO_2$ needle electrode for measurement in tissues | $pO_2$ catheter electrodes for measurement in blood vessels |
|---|---|---|
| overall length | 90 mm | 15 mm |
| tip length | 5—8 mm | — |
| tip diameter | 2—4 μm | 0.7—1.2 mm |
| diameter of the measurement surface | 0.5 μm | 15μm |
| membrane | collodium, polystyrol | 12 μm Teflon®, or collodium, polystyrol |
| sensitivity | $2 \times 10^{-12}$ A/Torr | $1 \times 10^{-11}$ A/Torr |
| drift | 2—5% | 1—3% |
| response time ($T_{90}$) | 0.8—1.2 sec | 2—3 sec |
| diffusion error | 1—5% | 3—6% |

Experimental procedure:

A $pO_2$— electrode was inserted in the muscle tissue (m. gastrocnemius) of both hindlegs of an anaesthetized cat. After recording the $pO_2$ of the normal perfused muscle, the blood flow to one measuring site was restricted by ligating the femoral artery. The $pO_2$ dropped steeply and the tissue became ischaemic. After a few minutes, the $pO_2$ increased and then decreased again. The values were constant after 30 to 60 min. After having reached a constant level of $pO_2$, the vehicle was given, followed by the test substance.

The recordings (Compound A i.v. vs. Compound B i.v.; Compound A i.d. vs. Compound B i.d., Compound C i.d.) were evaluated, taking one value every 10 sec — at least 36 readings being taken. The mean value and the standard deviation were calculated, in order to check the significance of the effect.

b) Skeletal muscle contractility:

Cats of either sex were anaesthetized as for $pO_2$ measurement. After dissection of the skin of the calf muscles, the sciatic nerve was cut about 3 cm proximal to the knee. The tendon of the calf muscles was cut and connected with an isometric force transducer (SWEMA, SG 3). In order to maintain constant differences and a resting tension of 100 p, the hind limb was fixed at the tibia by means of a clamp. Direct stimulation of the muscles consisted of square wave pulses of a 4 msec duration at a frequency of 2 Hz and at a voltage of 50 V. In order to keep the muscles wet and at a normal temperature, the muscles were continuously superfused by means of a NaCl-solution (0.9%, 38°C). Femoral blood flow was restricted by a graded occlusion of the artery leading to a reduction of contractility by ca. 30%. After having reached a constant level of the contraction force, the vehicle (NaCl and Methocel®, respectively) was injected, followed by the test substance.

Results:

Table 1 demonstrates that Compound A leads to a distinct increase of the contractility and $pO_2$ of ischaemic skeletal muscle. Surprisingly, Compound A shows high activity in the μg-range (50—125 μ/kg). In

contrast, the dose range of activity of Compounds B and C is 5—32 mg/kg, leading to changes which are less pronounced in comparison with Compound A (see tables 1—3) at 125 μg/kg.

TABLE 1

ISCHAEMIC MUSCLE — CAT

| | contractility (% initial value) | | $pO_2$ (mmHg) (increase) | |
|---|---|---|---|---|
| | i.v. | i.d. | i.v. | i.d. |
| Compound A (dose/kg) | 36 (50 μg) | 32 (125 μg) | 10 (125 μg) | 4 (50 μg) |
| Compound B (dose/kg) | 9 (5 mg) | 23 (32 mg) | 2 (5 mg) | 3 (12.5 mg) |
| Compound C (dose/kg) | NT | 22 (12.5 mg) | NT | 6 (32 mg) |

NT = not tested because of low water solubility

TABLE 2

COMPARISON COMPOUND B — COMPOUND A
$pO_2$ in skeletal muscle

Cat
1.8 — 2.6 kg
n = 6 ♂, ♀

| | $pO_2$ prior to ligation (Torr ± SEM)* | $pO_2$ after ligation (Torr ± SEM)* |
|---|---|---|
| hind limb art. ligation<br>control | 20.1 ± 0.04<br>11.0 ± 0.66 | 15.6 ± 0.19<br>— |
| hind limb art. ligation<br>control | 26.7 ± 0.08<br>12.0 ± 0.02 | 20.6 ± 0.52<br>— |
| hind limb art. ligation<br>control | 37.4 ± 0.1<br>— | 24.7 ± 0.64<br>— |
| hind limb art. ligation<br>control | 27.9 ± 0.99<br>20.1 ± 0.12 | 16.4 ± 1.14<br>— |
| hind limb art. ligation<br>control | 30.5 ± 0<br>2.0 ± 0 | 12.5 ± 0.12<br>— |
| hind limb art. ligation<br>control | 8.2 ± 0.23<br>13.71 ± 0.11 | 4.9 ± 0.23<br>— |

TABLE 2 — continued

| | effect of Compound B 5 mg/kg i.v. $\Delta pO_2$ (Torr) | effect of Compound A 125 µg/kg i.v. $\Delta pO_2$ (Torr) | effect of Compound B in relation to Compound A (%) |
|---|---|---|---|
| hind limb art. lig.<br>control | 1.6 ($p \leq 0.01$)<br>0.3 (n.s.) | 6.2 ($p \leq 0.01$)<br>13.2 ($p \leq 0.01$) | 26.6<br>0 |
| hind limb art. lig.<br>control | − 0.16 (n.s.)<br>1.02 ($p \leq 0.01$) | 7.3 ($p \leq 0.01$)<br>3.8 ($p \leq 0.01$) | 0<br>27 |
| hind limb art. lig.<br>control | − 1.5 (n.s.)<br>— | 5.2 ($p \leq 0.01$)<br>— | 0<br>— |
| hind limb art. lig.<br>control | 4.9 ($p \leq 0.01$)<br>− 2.4 ($p \leq 0.01$) | 27.1 ($p \leq 0.01$)<br>3.5 ($p \leq 0.01$) | 17.9<br>0 |
| hind limb art. lig.<br>control | 7.3 ($p \leq 0.01$)<br>14.3 ($p \leq 0.01$) | 11.2 ($p \leq 0.01$)<br>19.3 ($p \leq 0.01$) | 65.6<br>74.3 |
| hind limb art. lig.<br>control | 1.3 ($p \leq 0.01$)<br>− 0.9 ($p \leq 0.01$) | 5.6 ($p \leq 0.01$)<br>3.4 ($p \leq 0.01$) | 22.9<br>0 |

Mean effect of compound B at mg/kg compared to Compound A at 125 µ/kg = 21.3%
*n = between 36 and 120

TABLE 3

SUBSTANCE: Compound A
Formulation: aqueous solution

Species: Cat
Weight: 1.8—2.0 kg
n: 4 ♂, ±
Suppliers: Stock/Phillips

| | | Nacl-sol. | t (min) | dosage 50 µg/kg i.v. | t (min) |
|---|---|---|---|---|---|
| Δ muscle contract. | $\bar{X}$ ± S | ±0 | | +36.0*<br>±21.0 | >60 |
| Δ initial phase BP decrease | $\bar{X}$ ± S | ±0 | | −14.0*<br>± 8.0 | 2 |
| Δ second phase BP increase | $\bar{X}$ ± S | ±0 | | +17.0*<br>± 9.0 | 33 |

TABLE 3 — continued

| | | dosage<br>125 µg/kg i.v. | t<br>(min) | dosage<br>313 µg/kg i.v. | t<br>(min) |
|---|---|---|---|---|---|
| Δ muscle<br>contract. | $\bar{X}$<br>±<br>S | +19.0*<br>±12.0 | >60 | +25.0<br>n = 1 | >60 |
| Δ initial phase<br>BP<br>decrease | $\bar{X}$<br>±<br>S | −23.1*<br>± 3.3 | 3 | −25.0<br>n = 1 | 2 |
| Δ second phase<br>BP<br>increase | $\bar{X}$<br>±<br>S | +10.8<br>± 8.3 | 32 | +15.0<br>n = 1 | 6 |

(percentage of initial values) *$p<0.05$
(t = time interval to reach initial values)
The average decrease of muscle contractility induced by the arterial occlusion was 26%.
The $LD_{50}$ in mice of 1,3-di-n-butyl-7-(2-oxopropyl) xanthine has been found to be greater than 1 g/kg per oral.

**Claims**

1. The use of 1,3-di-n-butyl-7-(2-oxopropyl)xanthine for the manufacture of a medicament for increasing oxygen tension and contractility in ischaemic skeletal muscle.
2. The use of 1,3-di-n-butyl-7-(2-oxopropyl)xanthine for the manufacture of a medicament for the treatment of peripheral vascular disease.
3. The use as claimed in Claim 2 wherein the medicament is for the treatment of intermittent claudication.
4. The use as claimed in any preceding Claim, wherein the medicament comprises 1 to 30 mg of 1,3-di-n-butyl-7-(2-oxopropyl)xanthine and a pharmaceutically acceptable carrier therefor.
5. The use as claimed in any preceding Claim, wherein the medicament is adapted for administration by injection.
6. The use as claimed in Claim 5, wherein the medicament comprises an aqueous solution.
7. The use as claimed in any of Claims 1 to 4, wherein the medicament is adapted for oral administration.
8. The use as claimed in claim 7, wherein the medicament is in the form of a capsule or tablet.
9. The use as claimed in Claims 7 or 8, wherein the medicament weighs 80 to 600 mg.
10. The use as claimed in Claim 9 wherein the medicament weighs 100 to 400 mg.

**Patentansprüche**

1. Die Verwendung von 1,3-Di-(n-butyl)-7-(2-oxopropyl)-xanthin zur Herstellung eines Arzneimittels zum Erhöhen der chemischen Sauerstoffspannung und des Zusammenziehungsvermögens beim ischämischen Skelettmuskel.
2. Die Verwendung von 1,3-Di-(n-butyl)-7-(2-oxoprpyl)-xanthin zur Herstellung eines Arzneimittels zur Behandlung von Erkrankungen peripherer Gefässe.
3. Die Verwendung wie in Anspruch 2 beansprucht, wobei das Arzneimittel zur Behandlung von intermittierendem Hinken verwendet wird.
4. Die Verwendung wie in irgendeinem der vorstehenden Ansprüche beansprucht, wobei das Arzneimittel 1 bis 30 mg 1,3-Di-(n-butyl)-7-(2-oxopropyl)-xanthin und einen dafür pharmazeutisch verträglichen Träger umfaßt.
5. Die Verwendung wie in irgendeinem der vorstehenden Ansprüche beansprucht, wobei das Arzneimittel für eine Verabreichung durch Injizieren angepaßt ist.
6. Die Verwendung wie in Anspruch 5 beansprucht, wobei das Arzneimittel eine wässrige Lösung umfaßt.
7. Die Verwendung wie in irgendeinem der Ansprüche 1 bis 4 beansprucht, wobei das Arzneimittel für eine orale Verabreichung angepaßt ist.
8. Die Verwendung wie in Anspruch 7 beansprucht, wobei das Arzneimittel in der Form einer Kapsel oder Tablette ist.

9. Die Verwendung wie in Ansprüchen 7 oder 8 beansprucht, wobei das Arzneimittel 80 bis 600 mg wiegt.

10. Die Verwendung wie in Anspruch 9 beansprucht, wobei das Arzneimittel 100 bis 400 mg wiegt.

**Revendications**

1. Utilisation de la 1,3-di-n-butyl-7-(2-oxopropyl)xanthine pour la fabrication d'un médicament pour augmenter la pression d'oxygène et la contractilité dans un muscle ischémique du squelette.

2. Utilisation de la 1,3-di-n-butyl-7-(2-oxopropyl)xanthine pour la fabrication d'un médicament pour le traitement de maladie vasculaire périphérique.

3. Utilisation suivant la revendication 2, dans laquelle le médicament est pour le traitement de la claudication intermittente.

4. Utilisation suivant l'une quelconque des revendications précédentes dans laquelle le médicament comprend 1 à 30 mg de 1,3-di-n-butyl-7-(2-oxopropyl)xanthine et un support acceptable du point de vue pharmaceutique pour celle-ci.

5. Utilisation suivant l'une quelconque des revendications précédentes, dans laquelle le médicament est adapté à l'administration par injection.

6. Utilisation suivant la revendication 5, dans laquelle le médicament comprend une solution aqueuse.

7. Utilisation suivant l'une quelconque des revendications 1 à 4, dans laquelle le médicament est adapté à l'administration orale.

8. Utilisation suivant la revendication 7, dans laquelle le médicament est sous la forme d'une capsule ou d'un comprimé.

9. Utilisation suivant la revendication 7 ou la revendication 8, dans laquelle le médicament pèse 80 à 600 mg.

10. Utilisation suivant la revendication 9, dans laquelle le médicament pèse 100 à 400 mg.